# EUROPEAN PATENT SPECIFICATION

(11) **EP 3 592 239 B1**
(45) Date of publication and mention of the grant of the patent: **12.08.2020**
(21) Application number: 18716120.3
(22) Date of filing: 09.03.2018
(51) Int. Cl.: A61B 7/00

(54) **AUSCULTATION DEVICE**
AUSKULTATIONSVORRICHTUNG
DISPOSITIF D'AUSCULTATION

(30) Priority: 09.03.2017 EP 17160154
(43) Date of publication of application: 15.01.2020
(73) Proprietor: Koninklijke Philips N.V., 5656 AG Eindhoven (NL)
(72) Inventor: LUI, Wing, Lam, 5656 AE Eindhoven (NL); ROCCHI, Simona, 5656 AE Eindhoven (NL); ESCANO, SERRANO, Carmen, 5656 AE Eindhoven (NL); GEERLINGS, Alexander, Cornelis, 5656 AE Eindhoven (NL)
(74) Representative: Veligura, Alina Viktorivna
(86) International application number: PCT/EP2018/055986
(87) International publication number: WO 2018/162755

(56) References cited:
- CN-Y- 201 029 885
- CN-Y- 201 085 631
- US-A- 3 735 836
- Anonymous: "Stethoscope - Wikipedia", , 16 February 2017 (2017-02-16), XP055408469, Retrieved from the Internet: URL:https://en.wikipedia.org/w/index.php?t itle=Stethoscope&oldid=765735995 [retrieved on 2017-09-20]
- Philips: "Co-creating with the International Commitee of the Red Cross (ICRC)", You Tube, 12 April 2017 (2017-04-12), page 1, XP054978403, Retrieved from the Internet: URL:https://www.youtube.com/watch?time_con tinue=5&v=ltX6quWnSwQ [retrieved on 2018-06-07]

## Description

### FIELD OF THE INVENTION

This invention relates to an auscultation device.

### BACKGROUND OF THE INVENTION

Conventional Pinard horns are used by midwives and other trained healthcare professionals to detect the fetal heartbeat. Before positioning the Pinard horn, the trained healthcare professional normally carries out the Leopold maneuver to stimulate the fetus and enable easy detection. Through precise placement of the horn, the healthcare professional can calculate the direction of fetal heartbeat transmission and determine the fetal position. This information can be used to determine if the fetus is in a breech position or has any other issue.

The use of a Pinard horn requires specialist training. However, training can be inconvenient and ineffective since only one person at a time can listen through the horn. It is also hard for the trainer to describe the correct fetal heartbeat sound if the trainee and the trainer are not able to listen together. Switching between the trainer and trainee results in movement of the Pinard horn and changes in the pressure applied to the abdomen of the subject, resulting in inconsistency in the sound.

Listening to fetal movement and heartbeat can reassure expectant parents that the fetus remains healthy, thereby relieving pre-birth anxiety. Family members will struggle to distinguish relevant sounds not interpreted concurrently by a trained healthcare professional. Movement of the horn when it is passed between users could result in short-term loss of heartbeat detection, causing unnecessary anxiety to the family members. In the case of the pregnant subject, it is difficult, if not impossible, to achieve the correct position to listen to their unborn baby's movements given the rigid nature of a conventional Pinard horn.

Placing a microphone at the end of the Pinard horn instead of the healthcare professional's ear then connecting the microphone to a speaker or mobile application is a possible solution to the problem. However, such a solution is not practical. Performing the Leopold maneuver requires the healthcare professional to have both hands free whilst using their ear to hold the Pinard horn in place. Therefore, holding a microphone hinders performance of the Leopold maneuver whereas positioning a microphone close to the healthcare professional's ear may result in distortion of the sound or interference from noise produced by the healthcare professional. A microphone also requires a power supply, and there are some situations where this is not desired.

Several alternative auscultation devices are known in the art. Firstly, a standard stethoscope is used by doctors to listen to a subject's heartbeat. A standard stethoscope may be coupled to multiple headsets for example allowing multiple users to listen to sounds from within the abdomen. However, the in-built diaphragm of standard stethoscopes can interfere with the detection of fetal movement and heartbeat by inhibiting the optimal sound travel through the amniotic fluid and the subject's abdomen.

Fetal Doppler makes use of ultrasound technology, but the harm caused by prolonged exposure to ultrasound is unknown. A sonographer or highly trained healthcare professional is required to use the device and excessive sensitivity can result in high background noise making the fetal movement difficult to decipher. Neither sonographers nor stable electricity supplies are regularly available in many resource-limited environments where the cost of fetal Doppler detectors would also be prohibitively high.

CN 201085631Y discloses a fetal heart stethoscope with a single set of stereo headphones and an extendable clamp attached to the stethoscope head. Whilst the earpieces connect to the stethoscope head *via* separate tubes, they are not suitable to be used by more than one user.

CN 201029885Y discloses a medical stethoscope connected to multiple earpieces *via* rubber tubing. The flexible nature of the connection between the first earpiece and the end of the stethoscope head prevents the first user from using their ear to press the stethoscope head firmly and securely against the abdomen of the subject.

US 20030002685 A1 discloses an add-on part for an auscultation device which can include a wireless interface.

Therefore, there remains an unmet need for an auscultation device to detect fetal movement and heartbeat, which enables a second user to listen concurrently to fetal movement and is suitable for use in resource-limited environments.

### SUMMARY OF THE INVENTION

The invention is defined by the claims.

According to examples in accordance with an aspect of the invention, there is provided an auscultation device comprising:
a rigid hollow tubular body having an open first end portion adapted to be positioned on the abdomen of a subject and a second end portion opposite to the first end portion;
a first earpiece at the second end portion; and
a second earpiece mechanically and acoustically coupled to the second end portion by a flexible tube.

The first earpiece may simply be the end of the hollow tubular body, or else it may be a separate component which couples to the end of the hollow tubular body.

The hollow tubular body having an open first end portion adapted to be positioned on the abdomen of a subject and a second end portion opposite to the first end portion allows a first user to listen to fetal movement, as with a standard Pinard horn. The first user, who will normally be a trained healthcare professional, can use their ear to apply pressure to the second end portion or the first earpiece allowing them to securely hold the first end against the abdomen leaving their hands free to perform procedures such as inspecting the abdomen of the subject, performing the Leopold maneuver or identifying the position of the fetus. Such benefits are known to those skilled in the art.

The terms "rigid" and "flexible" are used as relative terms. It should be understood additionally that the "rigid hollow tubular body" is sufficiently rigid that it can be held in place between the ear of the (first) user and the abdomen of the subject in a hands-free manner. The "flexible tube" is sufficiently flexible that moved in position relative to the rigid hollow tubular body so that the tube can be positioned out of way and a second user who uses the second earpiece has some freedome of movement relative to the rigid hollow tubular body and hence also relative to the first user.

The addition of a second earpiece mechanically and acoustically coupled to the second end portion by a flexible tube allows a second user to listen to fetal movement concurrently without obstructing the movement of the first user. This means that the first user can describe the sounds coming from the subject's abdomen as they occur whilst maintaining the position and pressure of the hollow tubular body on the subject's abdomen.

Therefore, the invention allows the second user to hear the same sounds coming from the subject's abdomen and receive real-time interpretation of the sounds' meetings. This is important in a number of situations.

Firstly, the invention allows the first user to efficiently and precisely train the second user in the interpretation of sounds from the auscultation device, because there is no need for the two users to switch between a single earpiece. This removes uncertainty about whether the sounds remain constant over time. The invention also allows the second user to listen to the sounds coming from the subject's abdomen whilst the first user performs procedures such as inspecting the abdomen of the subject, performing the Leopold maneuver or identifying the position of the fetus.

Secondly, the invention allows expectant parents and their families to listen to fetal movements without the need to take control of the auscultation device. It thereby prevents anxiety if detection of fetal movement is temporarily lost.

Furthermore, where the second user is the pregnant subject, the flexible tube allows the subject to listen to the fetal movement in a way that is simply not possible with a standard, rigid Pinard horn.

The invention is particularly useful in resource-limited environments where the use of Doppler detectors is not a viable option due to their high cost, the lack of highlytrained sonographers and the absence of stable electricity supplies. The auscultation device of the invention will also not require regular servicing or calibration like Doppler detectors and stethoscopes.

The hollow tubular body preferably has a length from 5 cm to 30 cm.

This range of lengths of the hollow tubular body optimizes the space available for the first user to comfortably perform procedures and interact with the subject whilst allowing the first user to retain optimal control of the hollow tubular body when it is held against the subject's abdomen by the first user's ear. This range of lengths also optimizes the sound that reaches the earpiece of the first user.

The hollow tubular body may be a Pinard horn.

The shape of a standard Pinard horn is familiar to healthcare professionals and so will be most comfortable for them to use. It also removes the need for more specialist training for the auscultation device of the current invention beyond that needed to use a standard Pinard horn. The meaning of a "Pinard horn" is known to those skilled in the art.

The flexible tube may have a length from 10 cm to 100 cm.

This range of lengths of the flexible tube optimizes the space available for the second user to comfortably access the second earpiece without unduly restricting the movement of the first user. It also optimizes the sound that reaches the earpiece of the second user.

The first end portion of the hollow tubular body may comprise an add-on part including a microphone, a wireless communication unit and a battery.

This add-on part allows more sophisticated functionality, such as recording the fetal movement and sending it to a central system for analysis. The add-on will use much less power than a Doppler detector, so this feature is particularly useful in areas where there is no stable electricity supply. The add-on is also useful when the first user has only basic medical training as the sound can be sent for analysis by an expert, either in real time or at a later date. Therefore, this add-on part is particularly useful in resource-limited environments. The add-on has the further advantage that it does not prevent the primary user from using both hands to inspect the subject's abdomen. The positioning of the add-on at the first end of the hollow tubular body means it is located as far as possible from the first user, minimizing detection of unwanted sounds from the first user.

The auscultation device is preferably for use in listening to fetal movement, preferably fetal heartbeat.

"Fetal movement" is meant to include global movements (e.g. reorientation of the fetus within the uterus), external movements (e.g. movement of fetal limbs) and internal movements (e.g. fetal heartbeat). Whilst the auscultation device of the current invention could also be used to listen to any bodily noise that can be detected from a subject's abdomen, it is optimized for use on a pregnant subject to listen to fetal movement. It is particularly useful in detecting fetal heartbeat as it has optimal sensitivity to allow the users to hear the fetal heartbeat without picking up extraneous background noise.

Examples in accordance with another aspect of the invention provide a fetal monitoring method comprising:
placing a first end portion of an auscultation device which comprises a rigid hollow tubular body on the abdomen of the subject;
a first user placing an ear in contact with a first earpiece at a second end portion of the hollow tubular body;
the first user applying pressure to the first earpiece with their ear to hold the hollow tubular body in position; and
a second user to listening to fetal movement using a second earpiece which is mechanically and acoustically coupled to the hollow tubular body by a flexible tube.

### BRIEF DESCRIPTION OF THE DRAWINGS

Examples of the invention will now be described in detail with reference to the accompanying drawings, in which:
Figure 1 shows in schematic form the general components of a first example of an auscultation device;
Figure 2 shows the auscultation device without (top) and with (bottom) a first design of add-on part at the end of the hollow tubular body;
Figure 3 shows the auscultation device without (top) and with (bottom) a second design of add-on part within the hollow tubular body; and
Figure 4 shows the auscultation device in use.

### DETAILED DESCRIPTION OF THE EMBODIMENTS

The invention provides an auscultation device which comprises a rigid hollow tubular body with an open first end portion adapted to be positioned on the abdomen of a subject and a second end portion opposite to the first end portion. There is a first integrated earpiece at the second end portion, and a second remote earpiece coupled to the second end portion by a flexible tube.

This auscultation device allows a second user, such as a pregnant subject, to listen to fetal movement whilst the first user controls the device. The auscultation device does not require a stable mains power supply.

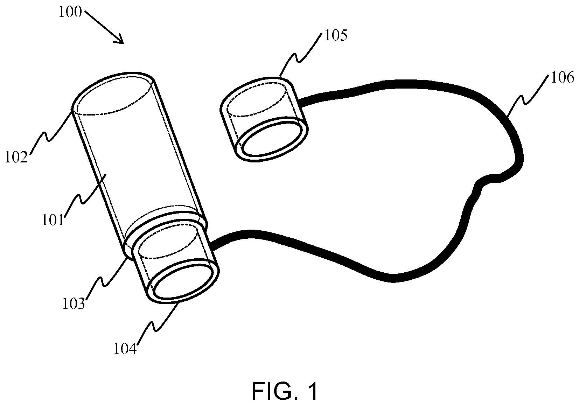

Figure 1 shows in schematic form the general components of the auscultation device 100 with dashed lines representing the internal surfaces of the rigid hollow tubular body 101, the first earpiece 104 and the second earpiece 105.

The auscultation device 100 comprises a hollow tubular body 101 through which sound is carried from an open first end portion 102, adapted to be positioned on the abdomen of a subject, to a second end portion 103 opposite to the first end portion 102. A first earpiece 104 is at the second end portion, and in particular is directly rigidly and acoustically coupled to the second end portion 103 to allow a first user to detect fetal movement. Furthermore, a second earpiece 105 is mechanically and acoustically coupled to the hollow tubular body 103 by a flexible tube 106. The second earpiece for example is coupled to the second end portion as well. However, it simply needs to couple the air pressure variations (i.e. the sound) within the hollow tubular body to the second earpiece, so it may in principle connect to any point along the hollow tubular body.

The first earpiece may be an integral part of the tubular body, and the flexible tube then simply connects to a lateral opening in that tubular body. Alternatively, and as shown, the flexible tube may attach to the first earpiece, which is itself separable from the hollow tubular body. The two earpieces then function as a connected pair, which is connected to the hollow tubular body as a unit.

The hollow tubular body 101 is shown as a cylindrical tube (i.e. with constant cross section). As is well-known, the tube may have a horn shape, with a relatively wide opening for pressing against the abdomen of the subject, and which narrows along its length towards the first earpiece 104. The first earpiece may then have a local wider portion. Any shape for the hollow tubular body is possible to provide the efficient travel of sound.

The hollow tubular body 101 may be made of any rigid material that allows it to be pressed firmly against the abdomen of the subject to form a good seal. Preferably the hollow tubular body 101 is made of metal, wood or plastic. More preferably the hollow tubular body 101 is made of plastic.

The first end portion 102 is open meaning that it does not contain a diaphragm or any other article that will seal the first end portion 102 and impede the travel of sound through the hollow tubular body 101. It is known to those skilled in the art that the abdomen of the subject can act as a diaphragm meaning sealing of the first end portion 102 can impede the travel of sound through the hollow tubular body 101.

The first end portion 102 is adapted to be positioned on the abdomen of a subject to allow a good seal to be formed between the first end portion 102 and the abdomen of the subject when gentle pressure is applied. The first end portion 102 is preferably smooth shape without sharp edges that may damage the skin of the subject.

The first earpiece 104 is rigidly and acoustically coupled to the second end portion 103. To facilitate cleaning and allow different earpieces to be attached, when a non-integrated first earpiece is used, it may be removable. When the first earpiece 104 is removable it may be attached to the second end portion 103 by any mechanical locking solution known to those skilled in the art including screwing or clipping.

The second earpiece 105 is mechanically and acoustically coupled to the second end portion 103 by a flexible tube 106. The second earpiece 105 can be directly or indirectly mechanically and acoustically coupled to the second end portion 103 by the flexible tube 106. Therefore, the flexible tube 106 can be directly attached to the hollow tubular body 101, to the second end portion 103 or to the first earpiece 104 so long as the flexible tube 106 mechanically and acoustically couples the second earpiece 105 to the second end portion 103 without unduly restricting the movement of the first user.

The flexible tube 106 that mechanically and acoustically couples the second end portion 103 to the second earpiece 105 can be made of any flexible, durable material that allows sound to travel unimpeded and is resistant to collapse. Preferably the flexible tube 106 is made of rubber. The flexible tube 106 may be of any diameter that allows sufficient sound to travel unimpeded to the second earpiece 105. The flexible tube 106 may be replaced so that the diameter of the flexible tube and material it is made from may be altered.

The hollow tubular body 101 for example has a length from 5 cm to 30 cm, more preferably from 10 cm to 25 cm and even more preferably from 15 cm to 22 cm.

The hollow tubular body may itself comprise a Pinard horn. In this case, an existing design of Pinard horn may be used, and the first and second earpieces then function as an extra component which converts the existing single earpiece into a double earpiece configuration. The first earpiece 104 may also form part of the Pinard horn for an integrated design. The shapes and dimensions suitable for use as a standard Pinard horn are known to those skilled in the art.

The flexible tube 106 for example has a length from 10 cm to 100 cm, more preferably from 20 cm to 90 cm and even more preferably 30 cm to 80 cm.

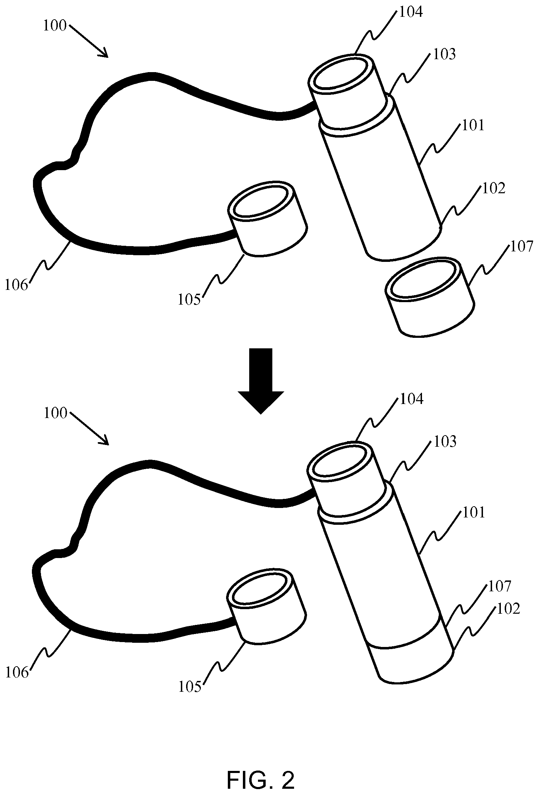

Figure 2 shows a second design of the auscultation device 100 without (top) and with (bottom) an add-on part 107 at the first end portion of the hollow tubular body 101.

The add-on part 107 includes a microphone, a wireless communication unit and a battery. The add-on part may be removable or semi-permanently attached. When the add-on part is attached to the first end portion of the hollow tubular body 101 as in Figure 2, it forms part of the hollow tubular body and then defines the open first end portion which is to make contact with the abdomen of the subject.

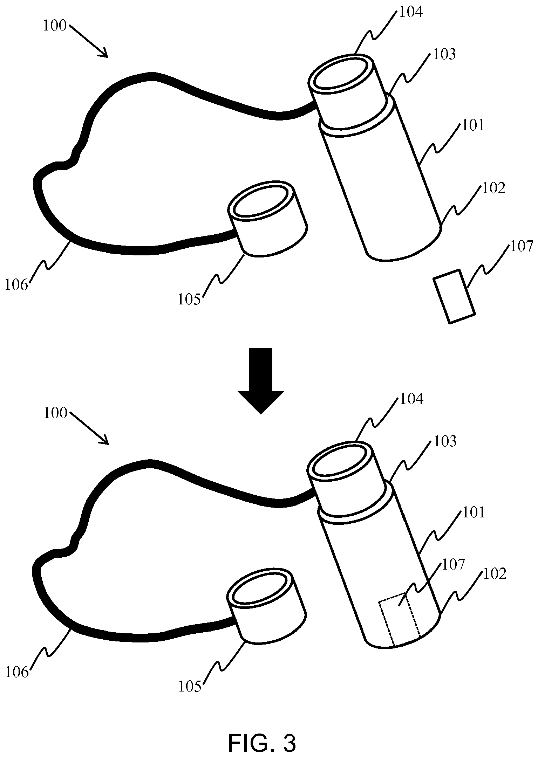

In an alternative embodiment shown in Figure 3, the add-on part 107 can be fitted the inside of the first end portion 102 of the hollow tubular body 101 using any suitable mechanical locking solution.

The wireless communication unit can communicate using wireless internet, Bluetooth, infrared or any other method known to those skilled in the art, for example to a portable mobile device such as a mobile telephone.

The auscultation device 100 is in particular for use in listening to fetal movement, especially the fetal heartbeat.

The examples above show only a single second earpiece 105, to minimize sound dilution and prevent tangling of the flexible tube 106. However, there may be third or further earpieces mechanically and acoustically coupled to the second end portion 103 by respective flexible tubes.

In some examples, the first ear piece 104 and second earpiece 105 are separable through space by a maximum distance of less than 200 cm, for example less than 150 cm or even less than 100 cm to allow the first and second user to more comfortably listen at the same time, but without excessively long tubes. The maximum distance is at least 30cm, for example at least 45cm.

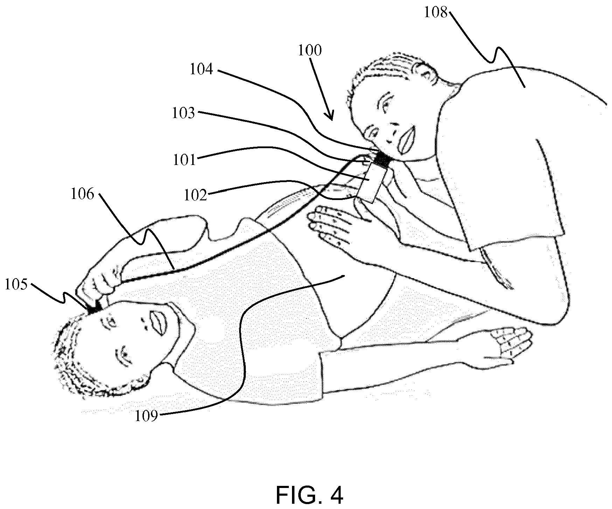

Figure 4 shows the auscultation device 100 in use with the open first end 102 of the hollow tubular body 101 positioned on the abdomen of a subject 109 and the first user 108 holding the hollow tubular body 101 in place by pressing their ear against the first earpiece 104, which is rigidly and acoustically coupled to the second end portion 103 of the hollow tubular body 101. In the example shown in Figure 4, the second user is the subject 109, who can listen to fetal movement through the second earpiece 105 mechanically and acoustically coupled to the second end portion 103 by the flexible tube 106.

However, it is understood that the second user can be the subject 109, a trainee healthcare professional, a trained healthcare professional or a family member of the subject. Preferably, the second user is a trainee healthcare professional or the subject 109.

The first user will generally be a trained healthcare professional such as a doctor, nurse or midwife. The first user may also be a trainee healthcare professional who is being supervised by a trained healthcare professional listening through the second earpiece 105.

The invention also provides a method of allowing a second user to listen to fetal movement detected by a first user 108 equipped with the auscultation device 100 described above.

The method comprises:
placing the first end portion 102 on the abdomen of the subject 109;
placing the ear of the first user in contact with the first earpiece 104;
applying pressure to the first earpiece 104 with the ear of the first user 108 to hold the hollow tubular body 101 in position;
optionally palpitating the abdomen of a subject 109 with both hands to stimulate the fetus;
optionally adjusting the position of the hollow tubular body 101; and
allowing a second user to listen to fetal movement using the second earpiece 105.

Palpitating the abdomen of the subject 109 with both hands to stimulate the fetus is optional when the fetus is already active and no manual inspection is required. Adjusting the position of the hollow tubular body 101 is optional when the correct position for optimum detection of fetal movement has been achieved.

All parts of the design may be created by 3D printing, injection molding or any other suitable methods. The add-on option is able to collect data for subsequent analysis, including noise cancellation.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. An auscultation device (100) comprising:
a rigid hollow tubular body (101) having an open first end portion (102) adapted to be positioned on the abdomen of a subject and a second end portion (103) opposite to the first end portion;
a first earpiece (104) at the second end portion; and
a second earpiece (105) mechanically and acoustically coupled to the hollow tubular body (101) by a flexible tube (106).

2. The auscultation device (100) according to claim 1 wherein the rigid hollow tubular body has a length from 5 cm to 30 cm.

3. The auscultation device (100) according to any preceding claim wherein the rigid hollow tubular body (101) comprises a Pinard horn.

4. The auscultation device (100) according to any preceding claim where the flexible tube (106) has a length between 30cm and 200 cm, for example between 45cm and 100cm.

5. The auscultation device (100) according to any preceding claim wherein the first end portion (102) of the rigid hollow tubular body (101) comprises an add-on part (107) including a microphone, a wireless communication unit and a battery.

6. A fetal monitoring system comprising the auscultation device (100) according to

7. A fetal monitoring method comprising:
placing a first end portion of an auscultation device (100) which comprises a rigid hollow tubular body (101) on the abdomen of the subject;
a first user placing an ear in contact with a first earpiece (104) at a second end portion (103) of the rigid hollow tubular body (101);
the first user applying pressure to the first earpiece (104) with their ear to hold the rigid hollow tubular body (101) in position; and
a second user listening to fetal movement using a second earpiece (105) which is mechanically and acoustically coupled to the rigid hollow tubular body (101) by a flexible tube (106).

## Patentansprüche

1. Auskultationsvorrichtung (100), umfassend:
einen starren hohlen röhrenförmigen Körper (101) mit einem offenen ersten Endabschnitt (102), der angepasst ist, um auf dem Bauch eines Subjekts positioniert zu werden, und einem zweiten Endabschnitt (103) gegenüber dem ersten Endabschnitt;
einen ersten Hörer (104) am zweiten Endabschnitt; und
einen zweiten Hörer (105), der mechanisch und akustisch durch ein flexibles Rohr (106) mit dem starren hohlen Rohrkörper (101) verbunden ist.

2. Auskultationsvorrichtung (100) nach Anspruch 1, wobei der starre hohle Rohrkörper (101) eine Länge von 5 cm bis 30 cm aufweist.

3. Auskultationsvorrichtung (100) nach einem vorhergehenden Anspruch, wobei der starre hohle Rohrkörper (101) ein Pinard-Rohr umfasst.

4. Auskultationsvorrichtung (100) nach einem vorhergehenden Anspruch, wobei das flexible Rohr (106) eine Länge zwischen 30 cm und 200 cm, beispielsweise zwischen 45 cm und 100 cm aufweist.

5. Auskultationsvorrichtung (100) nach einem vorhergehenden Anspruch, wobei der erste Endabschnitt (102) des starren hohlen Rohrkörpers (101) einen Zusatzteil (107) umfasst, der ein Mikrofon, eine drahtlose Kommunikationseinheit und eine Batterie enthält.

6. Fetales Überwachungssystem, umfassend die Auskultationsvorrichtung (100) nach Anspruch 5.

7. Fetales Überwachungsverfahren, umfassend:
Platzieren eines ersten Endabschnitts (102) einer Auskultationsvorrichtung (100), die einen starren hohlen röhrenförmigen Körper (101) umfasst, auf dem Bauch des Subjekts;
ein erster Benutzer, der ein Ohr an einem zweiten Endabschnitt (103) des starren hohlen Rohrkörpers (101) in Kontakt mit einem ersten Hörer (104) bringt;
der erste Benutzer, der mit seinem Ohr Druck auf den ersten Hörer (104) ausübt, um den starren hohlen Rohrkörper (101) in Position zu halten; und
ein zweiter Benutzer, der der fetalen Bewegung unter Verwendung eines zweiten Hörers (105) zuhört, der mechanisch und akustisch durch einen flexiblen Schlauch (106) mit dem starren hohlen Rohrkörper (101) verbunden ist.

## Revendications

1. Dispositif d'auscultation (100) comprenant:
un corps tubulaire creux rigide (101) ayant une première partie d'extrémité ouverte (102) adaptée pour être positionnée sur l'abdomen d'un sujet et une deuxième partie d'extrémité (103) opposée à la première partie d'extrémité;
un premier écouteur (104) à la deuxième partie d'extrémité; et
un deuxième écouteur (105) couplé mécaniquement et acoustiquement au corps tubulaire creux rigide (101) par un tube flexible (106).

2. Dispositif d'auscultation (100) selon la revendication 1, dans lequel le corps tubulaire creux rigide (101) a une longueur de 5 cm à 30 cm.

3. Dispositif d'auscultation (100) selon l'une quelconque des revendications précédentes dans lequel le corps tubulaire creux rigide (101) comprend une corne Pinard.

4. Dispositif d'auscultation (100) selon l'une quelconque des revendications précédentes où le tube flexible (106) a une longueur entre 30 cm et 200 cm, par exemple entre 45 cm et 100 cm.

5. Dispositif d'auscultation (100) selon l'une quelconque des revendications précédentes, dans lequel la première partie d'extrémité (102) du corps tubulaire creux rigide (101) comprend une partie complémentaire (107) comprenant un microphone, une unité de communication sans fil et une batterie.

6. Système de surveillance fœtale comprenant le dispositif d'auscultation (100) selon la revendication 5.

7. Procédé de surveillance fœtale comprenant:
la mise en place d'une première partie d'extrémité (102) d'un dispositif d'auscultation (100) qui comprend un corps tubulaire creux rigide (101) sur l'abdomen du sujet;
un premier utilisateur plaçant une oreille en contact avec un premier écouteur (104) au niveau d'une deuxième partie d'extrémité (103) du corps tubulaire creux rigide (101);
le premier utilisateur appliquant une pression sur le premier écouteur (104) avec son oreille pour maintenir le corps tubulaire creux rigide (101) en position; et
un deuxième utilisateur écoutant le mouvement du fœtus à l'aide d'un deuxième écouteur (105) qui est couplé mécaniquement et acoustiquement au corps tubulaire creux rigide (101) par un tube flexible (106) .
